# EUROPEAN PATENT APPLICATION

(11) **EP 2 431 461 A1**
(43) Date of publication of application: **21.03.2012**
(21) Application number: 10177742.3
(22) Date of filing: 20.09.2010
(51) Int. Cl.: C12N 9/20, C11D 3/386

(54) **Lipase congeners with enhanced substrate access**

(71) Applicant: Technische Universität Hamburg-Harburg, 21073 Hamburg (DE); TuTech Innovation GmbH, 21079 Hamburg (DE)
(72) Inventor: Budisa, Nediljko, 85622 Feldkirchen (DE); Wiltschi, Birgit, 79211 Dentzlingen (DE); Royter, Marina, 22081 Hamburg (DE); Antranikian, Garabed, 21218 Hittfeld-Waldesruh (DE); Grote, Ralf, 21075 Hamburg (DE)
(74) Representative: UEXKÜLL & STOLBERG

(57) **Abstract**

The present invention relates to the field of enzyme optimization, and in particular to the optimization of lipase enzymes. Specifically, the invention provides a lipase enzyme which has been modified by the incorporation of one or more non-canonical amino acids to give a lipase congener, said lipase congener having an increased enzymatic activity in an aqueous phase compared to the unmodified enzyme. The invention also relates to the use of such lipase congeners in the soap and detergent industry.

SEQUENCE LISTING

<110>  Technische Universität Hamburg-Harburg
       TuTech Innovation GmbH

<120>  Lipase congeners with enhanced substrate access

<130>  P 82490

<160>  3

<170>  PatentIn version 3.3

<210>  1
<211>  259
<212>  PRT
<213>  Thermoanaerobacter thermohydrosulfuricus

<400>  1

Met Gln Lys Ala Val Glu Ile Thr Tyr Asn Gly Lys Thr Leu Arg Gly
1               5                   10                  15

Met Met His Leu Pro Asp Asp Val Lys Gly Lys Val Pro Met Val Ile
            20                  25                  30

Met Phe His Gly Phe Thr Gly Asn Lys Val Glu Ser His Phe Ile Phe
        35                  40                  45

Val Lys Met Ser Arg Ala Leu Glu Lys Val Gly Ile Gly Ser Val Arg
    50                  55                  60

Phe Asp Phe Tyr Gly Ser Gly Glu Ser Asp Gly Asp Phe Ser Glu Met
65                  70                  75                  80

Thr Phe Ser Ser Glu Leu Glu Asp Ala Arg Gln Ile Leu Lys Phe Val
                85                  90                  95

Lys Glu Gln Pro Thr Thr Asp Pro Glu Arg Ile Gly Leu Leu Gly Leu
            100                 105                 110

Ser Met Gly Gly Ala Ile Ala Gly Ile Val Ala Arg Glu Tyr Lys Asp
        115                 120                 125

Glu Ile Lys Ala Leu Val Leu Trp Ala Pro Ala Phe Asn Met Pro Glu
    130                 135                 140

Leu Ile Met Asn Glu Ser Val Lys Gln Tyr Gly Ala Ile Met Glu Gln
145                 150                 155                 160

Leu Gly Phe Val Asp Ile Gly Gly His Lys Leu Ser Lys Asp Phe Val
                165                 170                 175

Glu Asp Ile Ser Lys Leu Asn Ile Phe Glu Leu Ser Lys Gly Tyr Asp
            180                 185                 190

Lys Lys Val Leu Ile Val His Gly Thr Asn Asp Glu Ala Val Glu Tyr
        195                 200                 205

Lys Val Ser Asp Arg Ile Leu Lys Glu Val Tyr Gly Asp Asn Ala Thr
    210                 215                 220

Arg Val Thr Ile Glu Asn Ala Asp His Thr Phe Lys Ser Leu Glu Trp
225                 230                 235                 240

Glu Lys Lys Ala Ile Glu Glu Ser Val Glu Phe Phe Lys Lys Glu Leu
                245                 250                 255

Leu Lys Gly

<210>  2
<211>  69
<212>  DNA
<213>  Artificial

<220>
<223>  PCR primer

<400>  2
tatatctata tcgaattcat taaagaggag aaattaagca tgcaaaaggc tgttgaaatt      60

acatataac                                                             69

<210>  3
<211>  76
<212>  DNA
<213>  artificial

<220>
<223>  PCR primer

<400>  3
cgtgccggcg gctgcagtta tcagtgatgg tgatggtgat gggatcctcc ctttaacaat      60

tcctttttga aaaact                                                     76

## Description

The present invention relates to the field of enzyme optimization, and in particular to the optimization of lipase enzymes. Specifically, the invention provides a lipase enzyme which has been modified by the incorporation of one or more non-canonical amino acids to give a lipase congener, said lipase congener having an increased enzymatic activity in an aqueous phase compared to the unmodified enzyme. The invention also relates to the use of such lipase congeners in the soap and detergent industry.

### BACKGROUND OF THE INVENTION

Lipases are glycerol ester hydrolases (EC 3.1.1.3.) which catalyze the cleavage of ester bonds in long-chain acylglycerols. Extracellular lipases from various microorganisms are widely used as technical enzymes for many important industrial applications. For example, microbial lipases are used in the food industry for the production of cheese and flavouring agents. Also, they are regularly employed as additives in soaps and washing powders.

However, lipase enzymes isolated directly from nature rarely exhibit the ideal combination of traits and activities required for the intended industrial use. Therefore, these enzymes must be optimized in order to exert their activity also in the particular non-natural environment that is defined by the industrial process for which they are used. A common problem experienced with the use of lipase enzymes is their inactivity in an aqueous environment. Without activation, lipases are normally not active in an aqueous phase, since the active site of the enzyme is covered in several lipases by an alpha-helical lid or flap structure composed of amino acids with amphiphilic properties (see Brzozowski et al. (1991), Nature, 351, 491; Vantilbeurgh et al. (1993), Nature, 362, 814). In the presence of lipid aggregates lipases show a sharp increase in their activity by the presence of a water/lipid interface, a phenomenon known as interfacial activation. Opening of the lid domain of a lipase in an aqueous phase can also be achieved by heating (thermal activation). At the structural level this process is characterized by a conformational rearrangement and movement ("opening") of a helical lid domain that covers the active site of the enzyme. In the closed conformation, the lid covers the enzyme active site, thereby blocking the access of substrate molecules. In contrast, the transition to the open conformation opens the entrance of the catalytic site for substrate molecules. The lid region of a lipase is normally an amphipathic protein domain with the hydrophilic side facing the solvent the hydrophobic side facing the protein core in the closed conformation. In the open conformation, the hydrophobic face becomes exposed and contributes to the substrate-binding region. Other lipases which do not possess a lid structure may include similar helical or non-helical protein domains which may interfere with the active site of the enzyme so as to block or inhibit the enzyme's activity in the aqueous phase.

In the prior art, lipases for industrial use have been modified by classical genetic approaches, such as site-directed mutagenesis, to remove the lid or flap structure so as to achieve constant substrate access to the active site even without initial thermal or interfacial activation. However, it has to be assumed that the lid structure that covers the active site of a lipase plays an important role in the overall structural integrity of the lipase, so that its removal might have detrimental effects on structural and functional integrity of the enzyme. Furthermore, the methods commonly used for site-directed mutagenesis are tedious and time-consuming.

Therefore, it has been an object of the present invention to provide modified lipase enzymes with enhanced substrate access and unimpaired structural and functional integrity. It has now been surprisingly found that lipase enzymes with such favourable characteristics can be provided by replacing certain amino acid residues, preferably in the lid region of the native lipase, by non-canonical amino acids. The modification can be achieved easily by methods that have been described in the prior art. The resulting lipase congeners are highly active without prior thermal or interfacial activation and at the same time have a primary structure which remains substantially unaltered compared to the native enzyme. Accordingly, this kind of modification avoids undesirable effects on integrity on enzymatic activity. The enhanced substrate access is of great advantage when using the lipases of the present invention in detergent applications, since the modified lipases are directly active even in the presence of small amounts of lipid aggregates which would normally not be sufficient to provide for an interfacial activation of the native lipase, or which would only result in a slow activation of the native lipase.

### DESCRIPTION OF THE INVENTION

The present invention relates in a first aspect to a lipase enzyme, preferably a lipase enzyme having a lid region or domain, which has been modified by the incorporation of one or more non-canonical amino acids, such that the resulting congenic enzyme has an increased enzymatic activity in an aqueous phase compared to the unmodified enzyme. This means that the incorporation of said non-canonical amino acid(s) results in an increase of activity of the modified congener relative to the unmodified lipase enzyme (i.e. the native lipase enzyme). The increase in lipase activity is preferably at least 2-fold, more preferably at least 3-fold, 4-fold, 5-fold, 6-fold, 8-fold, 10-fold or 15-fold, relative to the native lipase in the inactivated state. For the purpose of the present invention, the enzyme activity is referred to in units, wherein 1 unit (1 U) of lipase activity is defined as the amount of enzyme required for the conversion of 1 µmol substrate (e.g. pNPP) per minute under predefined conditions. The exact conditions (e.g., pH, temperature, salt concentration, etc.) to be applied in the activity measurements may vary to some extent depending on the enzyme employed. However, the skilled person will appreciate that different methods for determining the enzymatic activity may be used, as long as the native and the modified enzymes are analyzed under the same conditions. According to a preferred aspect of the invention, the enzymatic activity is determined at a temperature of 15 to 45°C, preferably 20-35°C, more preferably 25-30°C and/or in the presence of a concentration of less than 2 mM pNPP, more preferably less than 1 mM pNPP, preferably less than 0.5 mM pNPP, more preferably less than 0.2 pNPP, even more preferably less than 0.15 mM pNPP as substrate for the lipase.

The invention provides novel lipase enzymes which have modified to possess a permanently opened substrate access in the aqueous phase. As used herein, a lipase enzyme that has been modified by incorporation of one or more non-canonical amino acids is referred to as a "lipase congener" or a "congenic lipase".

The increase in activity of the congenic lipase can be observed when the enzyme is present in an aqueous phase, e.g. in an aqueous environment such as an aqueous solution. Preferably, the aqueous phase is comprised of at least about 50% v/v of water, more preferably about 60%, 70%, 80%, 85%, 90%, 95%, 96%, 97%, 98%, 99% v/v or more of water. In such an aqueous environment, the activity of a native lipase is usually low or absent, due to the helical lid domain which covers the tunnel-like active site of the enzyme. Activation of the lipase in the aqueous phase may be achieved either by heating the enzyme or by contacting it with a certain threshold concentration of lipid aggregates. Activation of native lipases by heating is normally achieved at a temperature of about 60-80°C, preferably 65-75°C, and more preferably 70°C for a time period of 60-120 minutes, preferably 90 minutes. For example, thermal activation is performed by heating the lipase for 10-60 minutes to 70°C. Accordingly, at ambient temperatures (i.e. at about 25°C) the native lipase enzyme is normally inactive or has a low activity in an aqueous phase, e.g. in an aqueous solution.

The lipases modified according to the invention have an increased activity in the absence of any prior thermal and/or interfacial activation. In other words, the modified lipases of the invention exhibit the increased enzyme activity at ambient temperature (i.e. at about 25°C), without prior heating of the enzyme. Likewise no prior contact with lipid aggregates is required to activate the enzyme. In contrast, the modified enzyme exerts its full activity upon the very first contact with lipids. Native lipases require a certain threshold level of lipid aggregates in the aqueous environment for full activation of the enzyme. This means that any concentration of lipids below the threshold level will not lead to activation so that no or insufficient conversion of the lipids occurs. The congenic lipases of the invention provide full enzymatic activity even at low lipid concentrations which do not trigger an interfacial activation.

The activity exerted by a lipase in the aqueous phase in the absence of thermal and/or interfacial activation can be determined, for example, as described in example 4 below. A substrate of the lipase enzyme, such as 4-nitrophenyl palmitate (pNPP), will be contacted in an aqueous solution, e.g. in 50 mM Tris-HCl pH 8.0, both with the unmodified lipase and the congenic lipase counterpart. Cleavage of the substrate is measured by suitable means, e.g. by using a spectrophotometer. The extent of the cleavage is determined and then correlated to the enzyme activity. In this way, the activity between an unmodified lipase and a congenic lipase derived therefrom can be compared. In this measurement, the concentration of the substrate is selected to be below the threshold level of interfacial activation, in the case of pNPP preferably below 1 mM, more preferably 0.15 mM.

In one embodiment of the invention, the native lipase enzyme has been modified by incorporating the one or more non-canonical amino acids into the lid region of the enzyme. However, since the folding of the helical lid domain is also influenced by amino acid residues which are located outside the lid domain, it is not obligatory that the incorporated non-canonical amino acid is located in the lid domain of the enzyme. The skilled person will readily be able to identify suitable amino acid positions outside the lid which could be used for increasing activity of the lipase enzyme.

For example, the incorporation of amino acid analogs in the region of the active site of the lipase may result in an amended activity profile of the enzyme. The active site of most lipases is buried inside the protein and contains a catalytic triad consisting of the amino acids serine, histidine, and aspartate (or glutamate). For example, the catalytic site of the lipase from *Thermoanaerobacter thermohydrosulfuricus* consists of the residues Ser113, Asp103 and His233, see SEQ ID NO:1. The skilled person is able to identify the catalytic triad in a given lipase based on the information available in the art.

As shown in the examples, the replacement of phenylalanine by fluorinated amino acid analogs was found to be suitable for increasing the activity of the lipase from *Thermoanaerobacter thermohydrosulfuricus* more than 2-fold.

However, it is particularly preferred that the incorporation of the non-canonical amino acid(s) occurs in the lid region.

The lid region of a lipase which covers the enzyme's active site can easily be identified, as it is well conserved among the group of lipases. In most cases, the lid region is a helical structure which interacts with the active site if the enzyme is in an aqueous medium. Accordingly, the skilled person is readily able to identify the location of the lid region within a given lipase sequence, e.g. by sequence comparison to well characterized lipase amino acid sequences available from public sequence databases. Another way to identify a lid region in a lipase enzyme is by software-based prediction of protein secondary and tertiary structure. Suitable software for predicting the secondary and/or tertiary structure of protein includes PHD (Rost et al. (1993), J. Mol. Biol. 232, 584-599), CABS (Kolinski (2004) Acta Biochimica Polonica, 51:349-371), Robetta (Kim et al. (2004) Nucleic Acids Research, 32, 526-31), I-Tasser (Yang Zhang (2008) BMC Bioinformatics, vol 9, 40) and the like.

From the methionine replacement experiments performed in the context of the present invention, it can be assumed that the enhanced substrate access which results in the increased activity of the modified lipases in aqueous medium is to be ascribed to an increase in the overall hydrophobicity of the lid region. As described in more detail below, moderately hydrophobic methionine residues were replaced by the extremely hydrophobic norleucine (Nle) so as to change the hydrophilicity/hydrophobicity balance in the lid region significantly. Thus, in one aspect, the invention contemplates modification of the lid region to increase the hydrophobicity of this protein domain by the incorporation of one or more non-canonical amino acids. In the context of the invention, the incorporation of one or more non-canonical amino acids can generally include both the incorporation of one or more additional non-canonical amino acids to a given native lipase sequence (so as to retain all of the canonical amino acids of the native enzyme) or the replacement of one or more canonical amino acids by appropriate non-canonical counterparts.

Preferably, the invention provides lipase enzymes having one or more methionine residues in the lid region in their unmodified native sequence which are then modified by replacement of said one or more methionine residues. The methionine residues are preferably replaced by a hydrophobic non-canonical amino acid such as norleucine (Nle).

Alternatively, the invention also refers to lipase enzymes which have been modified by the replacement of one or more native phenylalanine residues. Preferably, the one or more phenylalanine residues have been replaced by a fluorinated non-canonical amino acid, such as meta-fluorophenylalanine (mFF) or para-fluorophenylalanine (pFF).

As used in the context of the present invention, the term "canonical amino acid" refers to an amino acid which is encoded by one or more codons of the genetic code. These amino acids include alanine, leucine, isoleucine, lysine, methionine, asparagine, aspartic acid, cysteine, phenylalanine, glutamic acid, threonine, glutamine, tryptophan, tyrosine, serine, histidine, arginine, glycine, valine, and proline. The classical approach of generating protein variants is to replace certain canonical amino acids in the protein with other canonical amino acids using commonly known genetic engineering techniques, such as site-directed mutagenesis.

The invention, however, contemplates to modification of a lipase enzyme by co-translational incorporation of isosteric synthetic amino acids which are not encoded by the standard genetic code (referred to herein as "non-canonical amino acids"). These non-canonical amino acids differ from their canonical counterparts in that they include additional functional groups or moieties which are not present in the canonical amino acid. Non-canonical amino acids can be incorporated in a codon-specific manner which exploits the lack of absolute substrate specificity of aminoacyl-tRNA synthetases. Thus, non-canonical amino acids use the same routes for being incorporated into the target protein as their canonical counterparts. This means that non-canonical amino acids must have a shape and size which is similar to that of the natural amino acid to avoid rejection by the protein synthesis machinery.

Non-canonical amino acids which may be used for the replacement of canonical amino acids in a target protein include, for example, norleucine (Nle), azidohomoalanine (Aha), trans-4-fluoroproline (tFP), cis-4-fluoroproline (cFP), trans-4-hydroxyproline (tHP), cis-4-hydroxyproline (cHP), meta-fluorotyrosine (mFY), ortho-fluorotyrosine (oFY), meta-fluorophenylalanine (mFF), para-fluorophenylalanine (pFF). A comprehensive list of non-canonical amino acids which can be used in the present invention can be found in Budisa (2004), Angew. Chem. Int. Ed., 43, 6426.

Several methods have been described for the incorporation of non-canonical amino acids into the primary structure of a protein. For example, the method which is referred to in the literature as supplementation-based incorporation (SPI) uses an amino acid auxotrophic expression host which is supplemented with an amino acid analog during target protein expression. By exploiting the substrate tolerance of cellular uptake as well as the endogenous translation system, synthetic amino acids are translated into expressed proteins. This methodology allows the residue-specific replacement of a particular amino acid at all positions in the protein sequence without the need for DNA mutagenesis. For example, where it is intended to replace the methionine residues in a protein, the gene encoding such protein is expressed in a host cell (e.g. by use of an inducible expression vector that has been transferred to the host cells) which is auxotrophic with respect to methionine, i.e. unable to synthesize the methionine required for its growth. The host cells are initially cultured with a limited amount of methionine in the growth medium. After depletion of methionine in the medium which is indicated by a growth arrest, the cells are transferred to a medium which contains a non-canonical analog of methionine, e.g. norleucine (Nle). Only then expression of the target protein is induced which results in the global incorporation of the non-canonical analog of methionine into the target protein. The SPI method has been described extensively in several publications in the last decade (see, e.g., Minks et al. (2000), Tetrahedron, 56, 9431; Budisa (2004), Angew. Chem. Int. Ed., 43, 6426-6463). In the experiments of the invention described below, extensive protein modifications by the SPI method were performed on C-terminally hexahistidine-tagged TTL.

Another method which allows a site-specific, targeted incorporation of non-canonical amino acids has been described as "expanded genetic code" since it considers some termination triplets (e.g. amber) or quadruplets as blank codons for cellular code expansion. Synthetic amino acids are incorporated into single recombinant proteins by means of nonsense- or frameshift-suppression using genetically engineered components of the translational machinery (aminoacyl-tRNA synthetases, tRNAs, ribosomes and other components).

This method allows position-specific *in vivo* incorporation of non-canonical amino acids in response to in-frame UGA stop codon. This technique was developed by R. Furter (Protein Sci. 1998, 7, 419) who imported yeast PheRS/amber suppressor tRNA^{Phe} into the E. *coli* expression host. There is almost no cross-reactivity between the yeast and E. *coli* PheRS since identity elements in their tRNAs evolved in a completely different manner. Thus the basic prerequisite is the evolution of novel aminoacyl-tRNA synthetases (aaRSs) capable to specifically charge cognate tRNA intracellularly. Such aaRS:tRNA pair should be orthogonal, i.e. there should be no cross-reactivity between heterologous AARSs and tRNAs with the natural host endogenous synthetases, amino acids and tRNAs. Schultz and coworkers greatly improved this system by the discovery that M. *jannaschii* TyrRS:tRNA_{CUA}^{Tyr} orthogonal pair in E. *coli* provides a much better system for position-specific incorporation since both components of said pair exhibit greatly reduced cross-reactivity. This system proved to be especially useful for the incorporation of various non-canonical tyrosine and phenylalanine analogs or extended aromatic systems (see Xie and Schultz (2006), Nat Rev Mol Cell Biol, 7, 775-782).

The discovery of pyrrolysine (Pyl) which is dubbed as the "22^{nd} amino acid" opened great possibilities to incorporate a variety of amino acids with aliphatic side chains equipped with different chemical properties and functionalities. Pyl incorporation takes place as a natural nonsense suppression event without the need for specific sequence context (e.g. mRNA stem-loop structure). Furthermore, a specific synthetase (PylRS) which is capable of charging Pyl onto cognate suppressor tRNA^{Pyl} developed naturally. Pyl-tRNA^{Pyl} generated in this way is able to interact with bacterial elongation factors at the ribosome and participate in polypeptide synthesis. Engineered PylRS: tRNA^{Pyl} pairs were used for position-specific incorporation of versatile lysine analogues into proteins in single and multiple positions of target recombinant proteins (Namy et al. (2007), FEBS Lett., 581, 5282; Kaya et al. (2009), Chembiochem 10, 2858).

Generally, the lipase to be modified according to the invention can be obtained from different sources. The invention is not restricted to specific classes of lipases, so that enzymes of mammalian (e.g., human, bovine, canine, equine, feline, rat, murine, etc.), bacterial (e.g., from the genera *Pseudomonas, Bacillus, Acinetobacter, Corynebacterium, Pasteurella, Enterococcus, Lactobacillus, Propionibacterium, Proteus, Thermoanaerobacter,* etc.), or fungal (e.g., from the genera *Malassezia, Rhizopus, Thermomyces, Candida, Geotrichum,* etc.) origin may be used. In a particular preferred embodiment, the lipase is a bacterial lipase, more preferably a lipase from a thermophilic bacterium, e.g. from the genus *Thermoanaerobac*ter. In an even more preferred embodiment, the lipase is derived from the thermophilic anaerobic bacterium *Thermoanaerobacter thermohydrosulfuricus.* The amino acid sequence of the lipase of *Thermoanaerobacter thermohydrosulfuricus* is depicted in SEQ ID NO:1. This lipase can be readily expressed recombinantly in *E. coli.* It has broad substrate specificity and is resistant against various organic solvents and detergents. Accordingly, a lipase of the invention may comprise or consist of the amino acid sequence referred to herein as SEQ ID NO:1.

It will be appreciated that the modification described herein can also be performed on homologs of the lipase of *Thermoanaerobacter thermohydrosulphuricus.* For example, the invention is suitable for use with lipase enzymes from different species or strains of *Thermoanaerobacter* which may differ from SEQ ID NO:1 by a limited number of sequence deviations. Such lipase homologs can be derived, for example, from a bacterial genus that is closely related to *Thermoanaerobacter.*

Homologs of the sequence of SEQ ID NO:1 may, for example, differ from the sequence of SEQ ID NO:1 by deletion, substitution or addition of amino acids. Accordingly, one or more amino acids of the enzyme in SEQ ID NO: 1 may be substituted (by canonical or non-canonical amino acids) or deleted as long as the homolog resulting from the deletion or substitution is still an enzymatically active lipase, i.e. the modification should not substantially diminish the activity of the lipase. Generally, any amino acid residue of the amino acid sequences shown in SEQ ID NO:1 can be replaced by a different canonical or non-canonical amino acid, provided that the resultant sequence of the variant or congener is still an enzymatically active polypeptide with lipase function. In particular, the enzyme depicted in SEQ ID NO:1 may be modified by the substitution of a total of 1, 2, 3, 4, 5, 6, 7, 8, 9, 10, 15, or 20, amino acids of the enzyme depicted in SEQ ID NO:1.

If a lipase differs from SEQ ID NO:1 by one or more substitutions with other canonical amino acids, it is preferred that such substitutions are conservative substitutions, i.e. substitutions of one or more amino acid residues by a canonical amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Homologs of the lipase depicted in SEQ ID NO:1 will normally show a high degree of sequence identity with the sequence of SEQ ID NO:1. The amino acid identity will be at least about 70%, 75%, 80%, 85%, 90%, 91%, 92%, 93%, 94%, 95%, 96%, 97%, 98% or 99% when compared in an optimal alignment, for example, by the program BESTFIT using standard parameters. As used herein, a sequence identity of 90% means that 90% of the amino acids of an analyzed amino acid sequence stretch are identical to the sequence of the reference amino acid sequence depicted in SEQ ID NO:1. Methods and computer programs for determining amino acid sequence identity are well known in the art.

Likewise, polypeptides which differ from the sequence depicted in SEQ ID NO:1 by the insertion of one or more additional canonical or non-canonical amino acids are considered homologs in the context of the present invention. Such insertions can be made at any position of the polypeptide shown in SEQ ID NO:1. Likewise, homologs also include polypeptides in which one or more amino acids have been deleted relative to the polypeptide shown in SEQ ID NO:1. In principle, such deletions can be applied to any amino acid position of the sequence of SEQ ID NO:1.

Also encompassed by the invention are enzymatically active fragments of the lipase shown in SEQ ID NO:1 as well as enzymatically active fragments of the above-mentioned homologs of the lipase shown in SEQ ID NO:1. Enzymatically active fragments of the sequence shown in SEQ ID NO:1 or its homologs are polypeptides that differ from the amino acid sequence shown in SEQ ID NO: 1 (or from the respective homolog sequence) by the absence of one or more amino acids at the N-terminus and/or the C-terminus of the polypeptide. For example, a fragment of the sequence of SEQ ID NO: 1 may differ from the sequence of SEQ ID NO:1 by the lack of about 5, 10, 15, 20, or 25, 30, 35, 40, 45 or 50 amino acids at the N-terminus and/or the C-terminus, provided that such fragment retains at least a part of the enzymatic lipase activity of the original full-length enzyme depicted in SEQ ID NO:1. Likewise, a fragment of a homolog of SEQ ID NO:1 may differ from said homolog sequence by the lack of about 5, 10, 15, 20, 25, 30, 35, 40, 45 or 50 amino acids at the N-terminus and/or the C-terminus, provided that the fragment is still enzymatically active.

In a preferred aspect of the invention the lipase has been modified by the incorporation of one or more non-canonical amino acids into the lid region of the protein which covers the active site of the enzyme in its native state. For example, the lid region of the *Thermoanaerobacter* lipase set forth in SEQ ID NO:1 extends approximately from amino acids 140 to 183. In the examples described below, all 11 methionine residues which occur in the native sequence of the lipase of SEQ ID NO: 1 have been replaced by norleucine. Three of the methionine residues of the protein are located in the lid region, namely Met142, Met147 and Met158. While the residues Met147 and Met158 are surface exposed, residue Met142 is buried. The lipase from *Thermoanaerobacter* modified by the replacement of methionine by norleucine exhibits full activity in the aqueous phase in the absence of thermal or interfacial activation. To achieve such favourable activity profile, it may already prove sufficient to modify only the methionine residues Met142, Met147 and Met158 in the lid region, while the remaining 8 methionine residues remain unchanged. It is also conceivable that modification of only one or two of the methionine residues Met142, Met147 and Met158 in the lid region may be sufficient to increase the activity of the lipase in an aqueous phase without thermal or interfacial activation.

The reason for the improved activity of the Nle-TTL used in the examples in aqueous solution appears to be the increased hydrophobicity of the lid region. Methionine is a moderately hydrophobic amino acid with a solubility in water of about 0.33 M. In comparison, Nle has a solubility in water of about 0.12 M, which means that it is extremely hydrophobic and its presence at the lid region of the lipase could mimic hydrophobicity as delivered by lipid aggregates. In other words, by global replacement of Met by Nle, the hydrophilicity/hydrophobicity balance is changed so that permanent conversion of "closed" native TTL into "open" lipase variant could take place.

In a particularly preferred aspect, the invention provides the lipase enzyme of SEQ ID NO: 1 which has been modified by replacing all of the methionine residues in the native amino acid sequence of the protein by norleucine so that said lipase enzyme has an increased enzymatic activity in an aqueous phase in the absence of thermal or interfacial activation compared to the native enzyme.

The invention also relates to a method of preparing a lipase enzyme having an increased enzymatic activity in an aqueous phase compared to the unmodified lipase enzyme, comprising
(a) incorporating one or more non-canonical amino acids into the amino acid sequence of the lipase, preferably in the lid region of the lipase;
(b) obtaining the modified lipase enzyme.

As outlined above, the one or more non-canonical amino acids are preferably incorporated by replacing one or more methionine residues in the native enzyme. The methionine residues are preferably replaced by a hydrophobic non-canonical amino acid such as norleucine (Nle). Alternatively, the one or more non-canonical amino acids may be incorporated by replacing one or more native phenylalanine residues, e.g. by a fluorinated non-canonical amino acid, such as meta-fluorophenylalanine (mFF) or para-fluorophenylalanine (pFF).

The activity of a lipase prepared in this way may be increased at least 2-fold, preferably at least 5-fold or 10-fold relative to the native lipase.

Preferably, the enzyme prepared by the above method is derived from a thermophilic bacterium, more preferably from *Thermoanaerobacter thermohydrosulfuricus.* For example, the enzyme may comprise or consist of the sequence of SEQ ID NO:1 or an amino acid sequence which is at least 70% identical thereto.

In a preferred aspect, the method includes modification of the lid region of the lipase enzyme, preferably to increase the overall hydrophobicity of the lid region. For this purpose, one or more native methionine residues in the lid region may be replaced, preferably by a hydrophobic non-canonical amino acid such as norleucine (Nle).

In still a further aspect, the invention relates to the use of a modified lipase enzyme as described above for the cleavage of ester bonds in lipids, such as triglycerides, oils and fats. In particular, the modified enzymes of the invention may be used in industrial applications, such as in the preparation of food (e.g. cheese and yoghurt) and flavouring agents. In still a further aspect, the enzymes of the invention may be used as additives in soaps and/or detergents, such as dish washing machine detergents and laundry detergents. The use in laundry detergents, such as washing powders, is particularly preferred. Also, the modified lipases may be useful as catalysts for the resolution of racemic mixtures, such as racemic alcohols.

Finally, the invention relates to a soap and/or detergent composition comprising a lipase enzyme as described above.

### BRIEF DESCRIPTION OF THE FIGURES

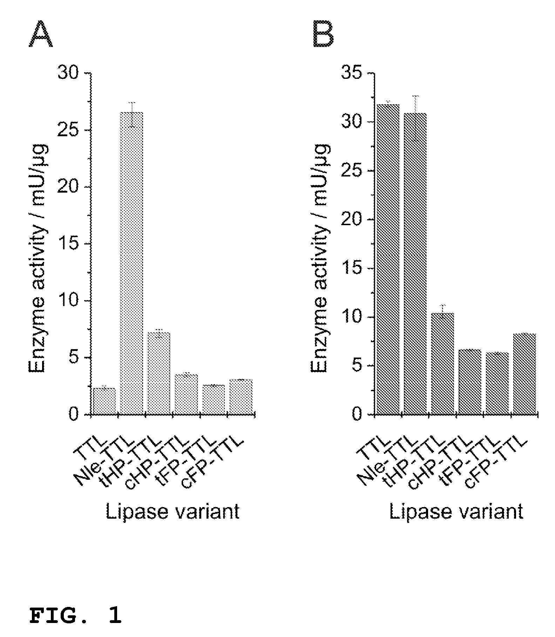

Fig. 1 shows the effect of heat activation on enzymatic activity by native and semisynthetic TTL variants containing Nle and fluorinated/hydroxylated Pro analogs. A: Activity levels without heat activation. Note that Nle-TTL exhibits about tenfold higher activity than parent TTL. B: Activity levels after heat activation. TTL is highly activated, Nle-TTL activity is unaffected and the activity of TTLs containing Pro analogs is only negligibly increased.

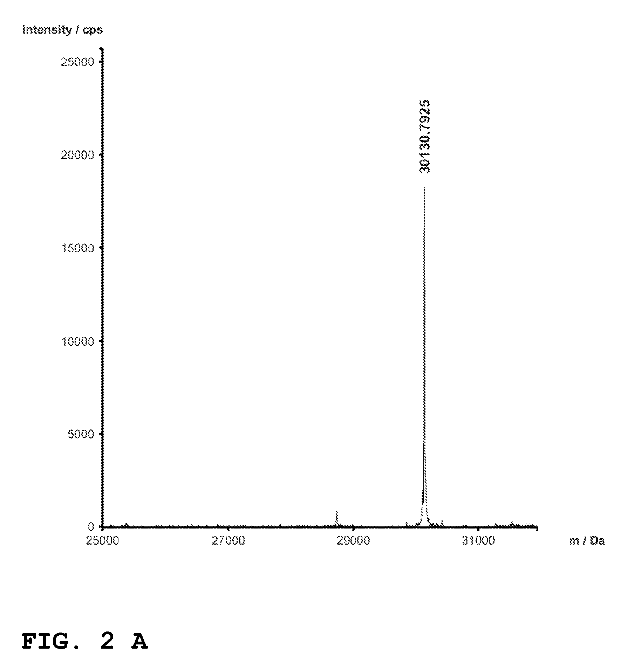

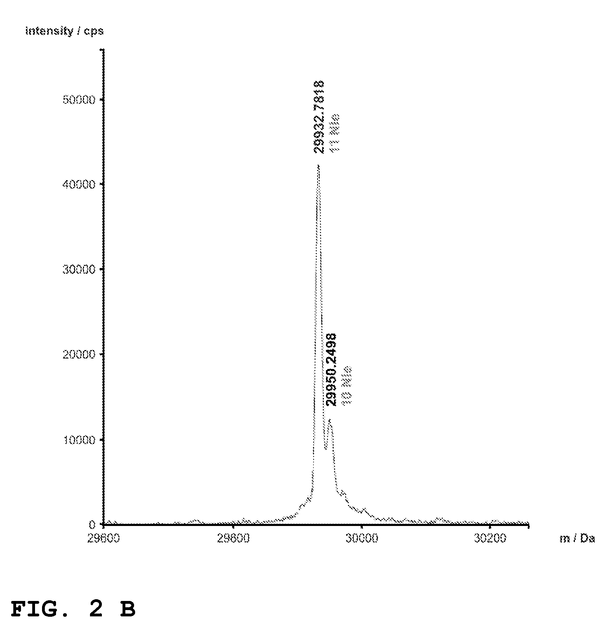

Fig. 2 shows the spectra resulting from electrospray ionization mass spectrometry (ESI-MS) of TTL (panel A) and the variant Nle-TTL (panel B).

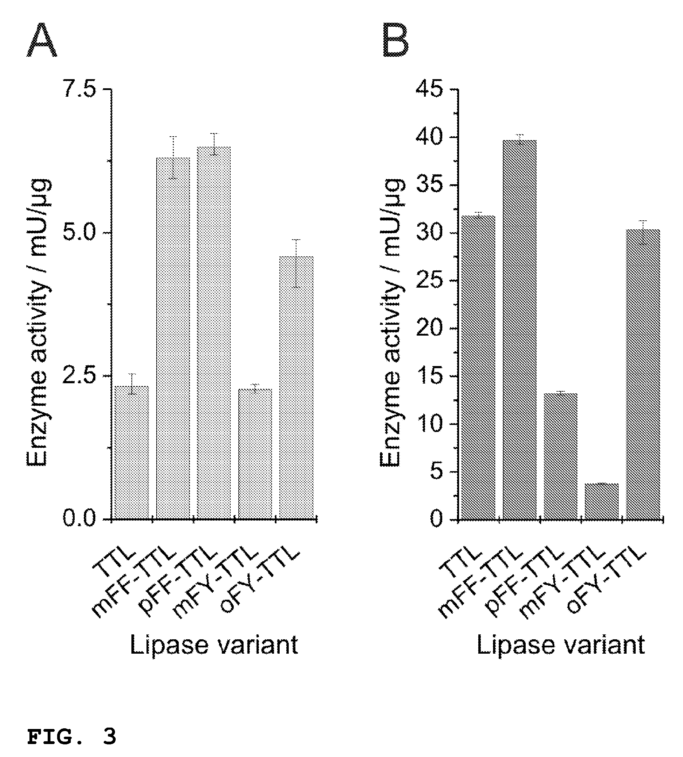

Fig. 3 shows the effect of heat activation on enzymatic activity by native and semisynthetic TTL variants containing aromatic phenylalanine analogs. A: Activity levels without heat activation. It can be seen that both mFF-TTL and pFF-TTL are more than 2-fold active in aqueous solution without heat activation when compared to native TTL. B: Activity levels after heat activation.

### EXAMPLES

All standard chemicals were obtained from Sigma (Steinheim, Germany) or Merck KGaA (Darmstadt, Germany) unless otherwise indicated. The L-isomers were used of all amino acids. Nle, tHP, cHP, mFF, pFF, and mFY were purchased from Sigma. The proline analogs tFP, and cFP were from Bachem AG (Bubendorf, Switzerland), and oFY was obtained from SynQuest Labs, Inc. (Alachua, FL). Trp analogs were prepared by enzymatic condensation of the corresponding indoles and L-serine with tryptophan synthase TrpS as described elsewhere (Bae et al. (2003) J Mol Biol., 328(5):1071-81). The reaction mixtures were added to the cells without further purification. 4-amino- and 7-azaindole were from Biosynth AG (Staad, Switzerland), and 4-fluoroindole was obtained from Molekula Deutschland (Vater-stetten, Germany).

The following E. *coli* strains were used for expression of TTL in the presence of different amino acid analogs: Met auxotrophic strain CAG18491 (LAM- rph-1 metEo-3079::Tn10) (CGSC *E. coli* Genetic Resources at Yale, CGSC# 7464) supplemented with Nle and Aha; Phe auxotrophic strain DG30 (proA2 aspC13 hisG4 ilvE12 argE3 thi-1 tyrB507 hsdS14 hppT29 lacY1 galK2 xyl-5 mtl-1 rpsL31 tsx-33 supE44 recB21 recC22 sbcB15 λ-) (*E. coli* Strain National BioResource Project, (NIG, Japan) strain no. ME8600) supplemented with mFF and pFF; Trp auxotrophic strain ATCC 49980 with the WP2 genotype (trp, uvrA, malB) supplemented with 4NW, 7AW, and 4FW; Pro auxotrophic strain CAG18515 (LAM- rph-1 proA3096::Tn10kan) (CGSC# 7331) supplemented with tHP, cHP, tFP, and cFP; and the Tyr auxotrophic strain AT2471 (LAM- e14- tyrA4 relA1 spoT1 thi-1) (CGSC# 4510) supplemented with mFY and oFY.

### EXAMPLE 1: Construction of expression plasmid pQE80L-TTL-H6

The sequence encoding TTL (SEQ ID NO:1) was PCR amplified with primers TTLpf (SEQ ID NO:2) and TTLpr (SEQ ID NO:3) obtained from Metabion International AG, Planegg-Martinsried, Germany. Primers TTLpf and TTLpr contain EcoRI and PstI cleavage sites flanked by additional random nucleotides at their 5'-ends for efficient restriction ("overhangs"). Primer TTLpr was designed such that a hexahistidine tag was attached to the C-terminus of TTL with an intervening short spacer (Gly-Ser). The PCR fragment was digested with EcoRI and PstI (New England Biolabs, Beverly, MA) and inserted into pQE80L (Qiagen, Hilden, Germany) cleaved with the same enzymes. The resulting expression plasmid pQE80L-TTL-H6 was sequence verified (DNA sequencing service at the Max Planck Institute of Biochemistry, Martinsried, Germany).

### EXAMPLE 2: Incorporation of non-canonical amino acids

The pQE80L-TTL-H6 expression vector was introduced into the different auxotrophic *E. coli* strains by electroporation following standard laboratory procedures. Plasmid-harboring clones were selected and propagated in media containing 100 mg/l ampicillin. For expression of TTL, the cells were first grown in New Minimal Medium (NMM) (Budisa et al. (1998), European Journal of Biochemistry 253, 1-9) supplemented with a limiting amount of Met, Phe, Pro, or Tyr.

After depletion of the canonical amino acid in the mid-log phase (OD₆₀₀ 0.5-0.8) as indicated by a growth arrest, the cells were shifted to NMM containing 0.5 mM either of the canonical amino acid (parent TTL expression) or a non-canonical analog (variant TTL expression). As outlined above, Trp analog synthesis batches were added to the cultures without further purification such that the final concentration of the corresponding indoles was 0.8 mM. For expressions involving the Pro analogs tHP and cHP, 500 mM NaCl was added to the cells 30 min prior to induction. Gene expression was induced by the addition of 1 mM isopropyl-β-D-1-thiogalactopyranoside (IPTG; Applichem, Darmstadt, Germany) and was performed for 4-5 h at 30°C with vigorous shaking.

### EXAMPLE 3: Mass analysis

For LC-ESI-MS, 20 µL aliquots of the purified variants were pre-separated on a Waters RP C4 column (300 Å pore size; 3.5 µm particle size; 100 x 2.1 mm; Waters GmbH, Eschborn, Germany) by eluting with a gradient from 20 to 90 % B in A within 20 min, where eluent A was 0.05 % (v/v) TFA in water and eluent B was 0.05 % (v/v) TFA in acetonitrile. A flow rate of 250 µl/min was used. The masses of the eluted fractions were analyzed on a MicroTOF ESI-MS (Bruker Daltonics, Bremen, Germany). Figure 2 shows the ESI-MS spectra of TTL (A) and the variant Nle-TTL(B).

### EXAMPLE 4: Activity assay with p-nitrophenyl palmitate

Lipase activity was determined by measuring the hydrolysis of p-nitrophenyl palmitate (pNPP, Sigma). Cleavage of pNPP was determined at the desired temperature in 50 mM Tris-HCl pH 8.0 according to Winkler and Stuckmann (J. Bacteriol. 1979, 138, 663). A buffered pNPP suspension (50 mM Tris-HCl pH 8.0, 1 mM pNPP, 1 mg/ml gum arabic (Acros Organics, Geel, Belgium)) was homogenized at 22,000 rpm using an Ultra-Turrax (IKA Werke GmbH & Co. KG, Staufen, Germany) for 4 min at room temperature. The reaction was started by mixing 900 µl buffered pNPP suspension with 100 µl of the TTL variant preparations.

The contribution of autohydrolysis was assessed by including a blank that contained the same volume of 50 mM Tris-HCl pH 8.0 instead of enzyme. The reaction mixture was incubated at the desired temperature for 10 or 15 min with vigorous shaking. Enzymatic hydrolysis of pNPP was stopped by addition of 100 µl of 1 M Na₂CO₃ and chilling on ice for 1 min. After spinning at 20,000 x g for 10 min at room temperature, the absorption of the supernatant was measured at 410 nm in a spectrophotometer (Ultrospec 6300 pro, Amersham Biosciences). All values were determined in triplicates and corrected for autohydrolysis. One unit (1 U) of lipase activity is defined as the amount of enzyme needed to liberate 1 µmol of pNPP (extinction coefficient ε_{pH} 8.0 = 12.75 x 10⁶ M⁻¹ cm⁻¹) per minute under the conditions described above.

The results of the activity assay are shown in Figure 1 and 3. It can be seen in panel A of Figure 1 that Nle-TTL exhibits about tenfold higher activity than parent TTL without heat activation. After heat activation, TTL is highly activated while Nle-TTL activity is unaffected (see panel B of Figure 1). As shown in Figure 3, similar results were obtained by incorporation of aromatic amino acid analogs into the native TTL. It can be seen that both mFF-TTL and pFF-TTL are more than 2-fold active in aqueous solution without heat activation when compared to native TTL.

## Claims

1. Lipase enzyme which has been modified by the incorporation of one or more non-canonical amino acids, wherein said modified lipase enzyme has an increased enzymatic activity compared to the unmodified lipase enzyme in an aqueous phase.

2. Lipase enzyme of claim 1, wherein said enzyme is derived from a thermophilic bacterium.

3. Lipase enzyme of claim 1 or 2, wherein said enzyme is derived from *Thermoanaerobacter thermohydrosulfuricus.*

4. Lipase enzyme of any of claims 1-3, wherein said enzyme comprises or consists of the sequence of SEQ ID NO:1 or an amino acid sequence which is at least 70% identical thereto.

5. Lipase enzyme of any of claims 1-4, wherein said modified enzyme has an activity that is increased at least 2-fold compared to the unmodified enzyme.

6. Lipase enzyme of any of claims 1-5, wherein said enzyme has been modified by incorporating one or more non-canonical amino acids in the lid region of the lipase enzyme.

7. Lipase enzyme of claim 6, wherein said enzyme has been modified to increase the overall hydrophobicity of the lid region.

8. Lipase enzyme of any of claim 6 or 7, wherein said enzyme has been modified by replacement of one or more native methionine residues in the lid region.

9. Lipase enzyme of claim 8, wherein the one or more methionine residues have been replaced by a hydrophobic non-canonical amino acid.

10. Lipase enzyme of claim 9, wherein the one or more methionine residues have been replaced by norleucine (Nle).

11. Lipase enzyme of any of claims 1-5, wherein said enzyme has been modified by replacement of one or more native phenylalanine residues.

12. Lipase enzyme of claim 11, wherein the one or more phenylalanine residues have been replaced by a fluorinated non-canonical amino acid.

13. Lipase enzyme of claim 12, wherein the one or more phenylalanine residues have been replaced by meta-fluorophenylalanine (mFF) or para-fluorophenylalanine (pFF).

14. Use of the lipase enzyme of any of claims 1-10 as an additive in soaps and/or detergents.

15. Use of the lipase enzyme of any of claims 1-10 in the food industry.

16. Soap and/or detergent composition comprising the lipase enzyme of any of claims 1-13.
